# EUROPÄISCHE PATENTANMELDUNG

(11) **EP 0 521 261 A1**
(43) Veröffentlichungstag der Anmeldung: **07.01.1993**
(21) Anmeldenummer: 92107839.0
(22) Anmeldetag: 09.05.1992
(51) Int. Cl.: A61B 1/00, A61B 1/12, A61B 17/36, A61B 17/39

(54) **Medizinisches Instrument mit einem Schalter zum Steuern externer Geräte**

(30) Priorität: 03.07.1991 DE 4121977
(71) Anmelder: Richard Wolf GmbH, D-75438 Knittlingen (DE)
(72) Erfinder: Heckele, Helmut, W-7134 Knittlingen (DE)
(74) Vertreter: Vollmann, Heiko, Dipl.-Ing.

(57) **Zusammenfassung**

Es ist ein medizinisches Instrument (1) mit einem Schalter (3) zur Steuerung des Betriebes eines über eine Steuerstrecke mit dem Schalter (3) in Wirkverbindung stehenden externen Gerätes (5) beschrieben. Bei diesem Instrument (1) wird die Steuerstrecke durch eine zwischen einer Lichtquelle und einem Lichtempfänger verlaufende Lichtstrecke (4) gebildet, wobei zwei Lichtleiter (12,13) Verwendung finden, die innerhalb des Instrumentes (1) so geführt sind, daß sich Ihre Enden mit Abstand gegenüberliegen. Zwischen den Enden dieser Lichtleiter (12,13) befindet sich eine Blende (11), die den von dem Lichtempfänger ankommenden Lichtstrom durch Betätigen des Schalters (3) derart verändert, daß durch zumindest das Ein- und Ausschalten des externen Gerätes (5) möglich ist.

## Beschreibung

Die Erfindung geht aus von einem medizinischen Instrument mit einem Schalter zur Steuerung des Betriebes eines über eine Steuerstrecke mit dem Schalter in Wirkverbindung stehenden externen Gerätes.

Bei endoskopischen Eingriffen werden häufig Arbeitsmedien eingesetzt, die zum großen Teil zeitweilig im Verlaufe des Eingriffs verfügbar sein müssen. Dazu finden entsprechende Zusatzgeräte, wie Hochfrequenz-Koagulationsgeräte, Lasergeräte, elektrisch betriebene Spül- und/oder Sauggeräte Verwendung, die meist über Fußschalter aktiviert oder deaktiviert werden. Allerdings sind auch zahlreiche Instrumente bekannt, bei denen entsprechende Schalter in die Instrumentenhandhabe oder den Instrumentengriff eingebaut sind.

So zeigt beispielsweise das DE-GM 1 840 499 einen in den Handgriff eines Resektoskops integrierten Schalter zum Öffnen bzw. Schließen des Hochfrequenz-Stromkreises. Die Betätigung des Schalters erfolgt mittels des Daumens der den Handgriff umspannenden Hand.

Nachteilig ist bei derartigen Schaltern, daß ihr Mechanismus kaum geeignet ist, den bei der Sterilisation der Instrumente auftretenden Sterilisationstemperaturen auf Dauer ohne Funktionsbeeinträchtigung zu widerstehen. Darüber hinaus besteht die Gefahr von Störungen durch in den Mechanismus eindringendes Wasser. Des weiteren liegt bei solchermaßen ausgeführten Instrumenten immer ein elektrisches Potential des Koagulationsgerätes an den Zuleitungen des Handgriffs und an den Schalterkontakten an, solange das Koagulationsgerät eingeschaltet ist. Es sind daher umfangreiche Maßnahmen zur elektrischen Isolation des Schalters and des Schaltergehäuses, also im Prinzip des Handgriffs, notwendig, um mögliche Gefährdungen des Bedienungspersonals and des Patienten auszuschließen.

Der Erfindung liegt daher die Aufgabe zugrunde, ein medizinisches Instrument mit einem handlich betätigbaren Schalter zum Schalten von bei endoskopischen Eingriffen eingesetzten Zusatzgeräten anzugeben, bei dem der Schaltermechanismus robust and unempfindlich gegenüber Temperatur- und Feuchtigkeitseinflüssen ist und eine Gefährdung des Benutzers and des Patienten durch unbeabsichtigten Stromübergang ausschließt.

Diese Aufgabe geht von dem einleitend angeführten Instrument aus and kennzeichnet sich weiter dadurch, daß die Steuerstrecke durch eine zwischen einer Lichtquelle und einem Lichtempfänger und teilweise durch das Instrument verlaufende Lichtstrecke gebildet ist, daß der Ausgang des Lichtempfängers den Gerätebetrieb steuert und daß mit dem Schalter der auf der Lichtstrecke beim Lichtempfänger ankommende Lichtstrom derart verändert ist, daß zumindest das Ein- and Ausschalten des Gerätes möglich ist.

Die damit erzielbaren Vorteile bestehen insbesondere darin, daß ein Energiemedium Verwendung findet, das für Benutzer and Patient gefahrlos handhabbar ist, und daß der Schaltermechanismus den auftretenden physikalischen Beanspruchungen gegenüber unempfindlich ist.

Weitere vorteilhafte Weiterbildungen des Erfingungsgegenstandes sind in den Unteransprüchen aufgeführt.

Die Erfingung ist nachstehend anhand des in der Zeichnung dargestellten Ausführungsbeispiels näher erläutert. Es zeigen:
- Figur 1: eine schematische Darstellung einer Anordnung aus Zusatzgerät und Instrument mit erfindungsgemäßem Schalter,
- Figur 2: eine schematische Schnittdarstellung eines erfindungsgemäßen Schalters nach einer ersten Ausführungsform in geschaltetem Zustand,
- Figur 3: eine vergrößerte Darstellung des Schalters nach Figur 2 in teilweisem Schnitt,
- Figur 4: eine vergrößerte Darstellung der Blende in einer Alternativausführung.

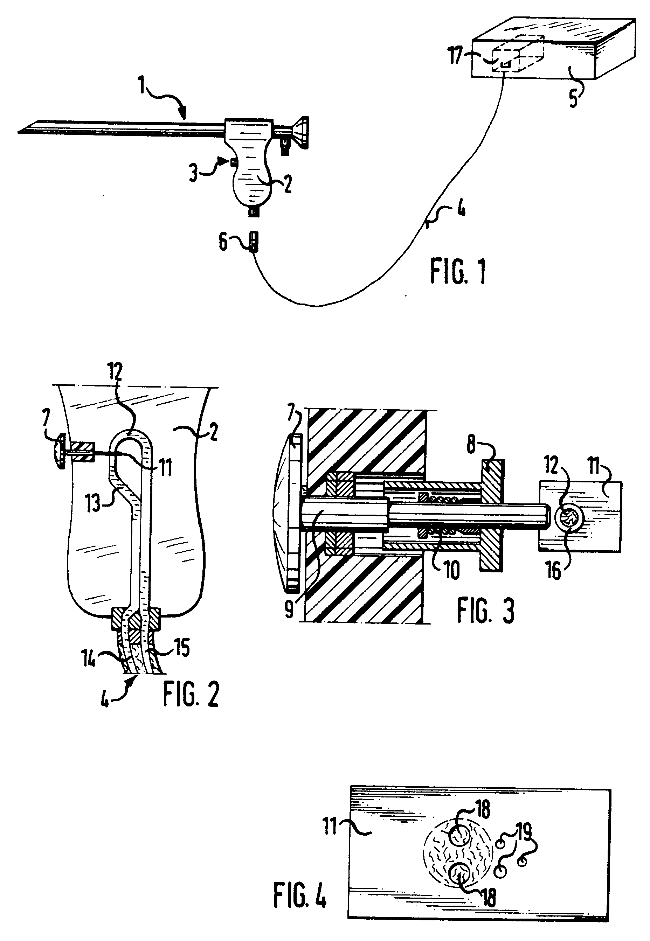

Der für medizinische Instrumente gedachte Schalter 3 ist, wie in Figur 1 angedeutet, beispielsweise in den Handgriff 2 eines Endoskops 1 integriert. Dieses steht über ein flexibles Lichtleitkabel 4 mit einem Zusatzgerät 5 in Verbindung, das z. B. ein elektrisch betriebenes Spül- oder Sauggerät sein kann. Das Lichtleitkabel 4 kann mittels einer Steckverbindung 6 an dem Endoskop 1 angekoppelt sein.

Der Schalter 3 umfaßt nach Figur 3 einen Betätigungsknopf 7, der in einer Halterung 8 mittels eines Schaftteils 9 längsbeweglich geführt ist. Dabei bewirkt eine Feder 10, daß der Betätigungsknopf 7 unbetätigt in ausgeschobener Stellung verharrt. Der Schaftteil 9 steht an seinem dem Betätigungsknopf 7 abgewandten Ende mit einer flächigen Blende 11 in Verbindung, die daher durch Betätigen des Betätigungsknopfes 7 im Sinne der Bewegung desselben Verschiebbar ist. Die Blende 11 ist dabei in einer Ebene gefühurt, zu deren beiden Seiten jeweils ein Lichtleiter 12 bzw. 13 endet und zwar so, daß sich diese Enden einander genau gegenüberliegen. Die den besagten Enden zugeordneten Lichtleiter 12 und 13 sind handgriffseitig an der Steckverbindung 6 angeschlossen und werden in dem Lichtleitkabel 4 also Zuleiter 14 und Rückleiter 15 weitergeführt. Die Blende 11 besteht aus einem lichtundurchlässigen Material und weist einen lichtdurchlässigen Bereich 16 auf, der durch Einschieben des Betätigungsknopfes 7 zwischen die Enden der Lichtleiter 12 und 13 gelangt.

Das Zusatzgerät 5 umfaßt neben der Pumpeneinrichtung eine Steuereinheit 17, die im wesentlichen eine Lichtquelle und einen Lichtempfänger sowie eine geeignete Auswerteschaltung für das empfangene Lichtsignal enthält. Das Lichtleitkabel 4 ist an dem Zusatzgerät 5 so angeschlossen, daß der Zuleiter 14 der Lichtquelle und der Rückleiter 15 dem Lichtempfänger zugeordnet ist.

Die Wirkungsweise des mit dem Beschriebenen Schalter versehenen Instruments ist folgende:
Mit Einschalten der Steuereinheit 17 gelangt Licht aus dem Lichtsender dieser Einheit über den Zuleiter 14 (Figur 2) und den in den Handgriff 2 verlaufenden Lichtleiter 13 an das neben der Blende 11 befindliche Ende desselben. In Ruhestellung des Schalters 3 ist dieser Teil der Blende 11 lichtundurchlässig, so daß der Lichtstrahl hier endet. Durch Eindrücken des Betätigungsknopfes 7 wird nun bewirkt, daß die Blende 11 sich mit ihrem lichtdurchlässigen Bereich 16 vor das besagte Ende des Lichtleiters 13 bewegt (Figur 3), so daß der Lichtstrahl in das gegenüberliegende Ende des Lichtleiters 12 eintreten und damit über das Lichtleitkabel 4 in den Lichtempfänger in der Steuereinheit 17 gelangen kann. Das dort empfangene Signal wird in für den entsprechenden Fachmann geläufiger Art und Weise durch die Steuereinheit 17 so verarbeitet, daß das Zusatzgerät 5 in einer bevorzugten Schaltung der Steuereinheit 17 bei Empfang einer Lichtmenge durch den Lichtempfänger eingeschaltet wird, während bei Ausbleiben des Lichtsignals am Lichtempfänger das Zusatzgerät 5 ausgeschaltet wird.

Nach einer weiteren Ausgestaltung der Erfindung ist es auch möglich, den lichtdurchlässigen Bereich 16 so auszubilden, daß nicht nur die Schaltzustände "aus" und "ein" möglich sind, sondern auch lichtmengenabhängige Schaltungen durchgeführt werden können. Dazu kann die Blende 11 entsprechend Figur 4 in Bereiche aufgeteilt werden, die eine kleinere oder größere Lichtmenge passieren lassen können. Dies kann beispielsweise dadurch geschehen, daß die Blende 11 mit Öffnungen 18 bzw. 19 verschiedener Größe versehen wird. Zu diesem Zweck muß der Schalter 1 entsprechend verschiedene Schalterstellungen zulassen, die für den Bediener unterscheidbar sind. Mit Hilfe dieser Ausführungsvariante des Schalten 1 besteht die Möglichkeit der Regelung von Zusatzgeräten 5 in Abhängigkeit von der jeweils an dem Lichtempfänger ankommenden Lichtmenge oder es können verschiedene Zusatzgeräte 5 lichtmengenabhängig ein- und ausgeschaltet werden. Weiter ist auch eine Kodierung der Blende 11 durch bestimmte Anordnung der Öffnungen 18 und 19 denkbar, wodurch ebenfalls verschiedene Zusatzgeräte 5 auch gleichzeitig schaltbar wären.

## Patentansprüche

1. Medizinisches Instrument mit einem Schalter (3) zur Steuerung des Betriebes eines über eine Steuerstrecke mit dem Schalter (3) in Wirkverbindung stehenden externen Gerätes (5), dadurch gekennzeichnet, daß die Steuerstrecke durch eine zwischen einer Lichtquelle und einem Lichtempfänger und teilweise durch das Instrument (1) verlaufende Lichtstrecke gebildet ist, daß der Ausgang des Lichtempfängers den Gerätebetrieb steuert und daß mit dem Schalter (3) der auf der Lichtstrecke beim Lichtempfänger ankommende Lichtstrom derart veränderbar ist, daß zumindest das Ein- und Ausschalten des Gerätes (5) möglich ist.

2. Instrument nach Anspruch 1, dadurch gekennzeichnet, daß die Lichtstrecke mit Lichtleitern (14 und 15) gebildet ist, die in das Instrument (1) eingeführt sind, wobei sich ihre dort befindlichen Enden mit Abstand gegenüberliegen, während das andere Ende des einen Lichtleiters auf die Lichtquelle und das andere Ende des anderen Lichtleiters auf den Lichtempfänger ausgerichtet ist, und daß mit dem Schalter (3) eine im Bereich zwischen den sich gegenüberliegenden Lichtleiterenden wirksame Blende (11) zwecks Veränderung des Lichtstromes verstellbar ist.

3. Instrument nach Anspruch 2, dadurch gekennzeichnet, daß die Blende (11) mit einem Betätigungsknopf (7) verbunden ist, der an einem Schaftteil (9) längs beweglich geführt gegen die Kraft einer Feder (10) eindrückbar ist.

4. Instrument nach Anspruch 2 oder 3, dadurch gekennzeichnet, daß die Blende (11) mindestens einen lichtdurchlässigen und einen lichtundurchlässigen Bereich (16 bzw. 18 und 19)aufweist.

5. Instrument nach einem der Ansprüche 2 bis 4, dadurch gekennzeichnet, daß die Blende (11) in der durch die Feder (10) erzeugten Ruhestellung des Schalters (3) so ausgerichtet ist, daß der Lichtstrom in dem Lichtleiterkabel (4) unterbrochen ist.

6. Instrument nach einem der Ansprüche 2 bis 5, dadurch gekennzeichnet, daß die Blende (11) Bereiche unterschiedlicher Lichtmengendurchlässigkeit aufweist.

7. Instrument nach Anspruch 6, dadurch gekennzeichnet, daß die Blende (11) mehrere Öffnungen (18 bzw. 19) verschiedener Größe aufweist.
